# EUROPEAN PATENT SPECIFICATION

(11) **EP 2 185 128 B1**
(45) Date of publication and mention of the grant of the patent: **08.08.2018**
(21) Application number: 08755460.6
(22) Date of filing: 14.05.2008
(51) Int. Cl.: A61K 8/34, A61Q 1/08

(54) **COSMETIC COMPOSITIONS CONTAINING RESVERATROL DERIVATIVES**
KOSMETISCHE ZUSAMMENSETZUNGEN MIT RESVERATROL-DERIVATEN
COMPOSITIONS COSMÉTIQUES CONTENANT DES DÉRIVÉS DE RESVÉRATROL

(30) Priority: 31.07.2007 US 952874 P; 31.07.2007 US 952877 P; 31.07.2007 US 952878 P; 31.07.2007 US 952881 P; 31.07.2007 US 952884 P
(43) Date of publication of application: 19.05.2010
(73) Proprietor: ELC Management LLC, New York, NY 10153 (US)
(72) Inventor: MAES, Daniel, Huntington, New York 11743 (US); MOHAMMADI, Fatemeh, Hauppauge, New York 11788 (US); CZARNOTA, Anna, Huntington, New York 11746 (US); MAMMONE, Thomas, Farmingdale, New York 11735 (US); DECLERCQ, Lieve, B-2180 Ekeren (BE); QU, Lisa, Flushing, New York 11354 (US); ZECCHINO, Jules, Closter, New Jersey 07624 (US)
(74) Representative: Dehns
(86) International application number: PCT/US2008/063610
(87) International publication number: WO 2009/017866

(56) References cited:
- WO-A1-2006/029484
- WO-A1-2006/134282
- WO-A2-99/04747
- WO-A2-2005/069998
- US-A1- 2002 051 799
- US-A1- 2007 015 840
- US-B1- 6 358 517
- US-B1- 6 572 882

## Description

### Technical Field

The invention is in the field of cosmetic compositions for application to keratinous surfaces such as skin, hair, or nails, for purposes of coloring or treating the surface. The term "cosmetic" when used herein means topical compositions that are applied keratinous surfaces for cosmetic or pharmaceutical purposes.

### Background of the Invention

Resveratrol, also referred to as 3,5,4'-trihydroxystilbene, is a polyhydroxy- substituted compound having the general formula:

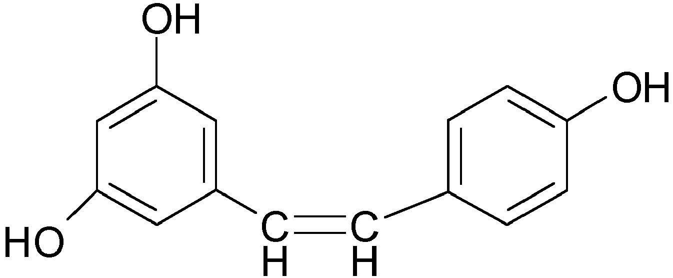

It is present in red grapes, raspberries, blueberries, and certain other plant berries or extracts. It has been reported that resveratrol has anti-aging, anti-cancer, and antiviral effects. Because of its perceived fountain-of-youth properties, resveratrol has been incorporated into a variety of cosmetic formulations, such as skin creams. However, one problem with resveratrol is that it is generally unstable in cosmetic formulations. Accordingly, if used in cosmetic formulas, it can only be used in very small amounts. If present in too large an amount, the resveratrol will hydrolyze and cause the cosmetic formulation into which it is incorporated to become discolored.

One way to address the instability of resveratrol is to derivatize the resveratrol by substituting the hydroxy groups at the 3, 5, and 4' position with other functional groups to form resveratrol derivatives that are more stable in cosmetic formulas. It has been discovered that resveratrol derivatives of inorganic acids, organic carboxylic acids, mono-, di-, or polysaccharides, or other functional groups are more stable in cosmetic formulations, particularly those in the emulsion form.

WO 2006/029484 describes topical compositions containing phosphorylated polyphenols. Example 5 illustrates a formulation comprising resveratrol triphosphate.

It is an object of the invention to provide an aqueous based cosmetic composition containing at least one resveratrol derivative, preferably one that is hydrolytically stable in cosmetic formulations that are aqueous based.

### Summary of the Invention

The invention is directed to an emulsion cosmetic composition comprising at least one resveratrol derivative, an aqueous phase, and an oil phase containing at least one oil phase structuring agent.

### Detailed Description

The compositions of the invention may be in emulsion form, such as water-in-oil or oil-in- water. In the emulsion form, the amount of water may range from about 0.1-99%, preferably from about 5-85%, more preferably from about 7-75% by weight of the total composition. In the emulsion the amount of oil will preferably range from about 1-95%, preferably from about 5-85%, more preferably from about 7-65% by weight of the total composition.

### RESVERATROL DERIVATIVES

Without being bound by this explanation, it is believed that the practical use of resveratrol in cosmetic compositions has been limited by instability due to the phenol groups. The resveratrol derivatives used in the compositions of the invention contain protective groups, which function to stabilize the phenol groups of resveratrol and make the resveratrol derivative suitable for use in emulsions where superior aesthetics and stability are required for commercially acceptable products. In addition, once the compositions containing the derivative is applied to skin, the protective groups can be easily hydrolyzed from the molecule, preferably by enzymes and other ingredients on the skin surface, to release an active form of resveratrol into the skin.

The resveratrol derivatives of the present invention have a general formula of: 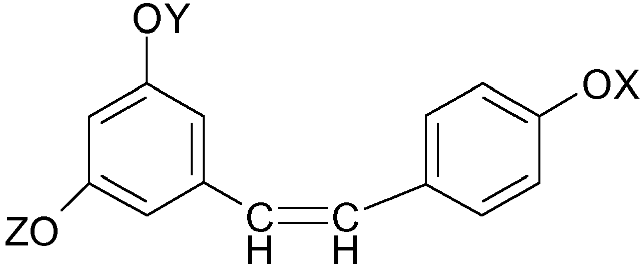 wherein X, Y, and Z are either hydrogen or a protective group, provided that at least one of X, Y, and Z is the protective group.

The resveratrol derivatives may be present ranging from about 0.001 to 95%, preferably from about 0.005 to 90%, more preferably from about 0.1 to 20% by weight of the total composition.

### Resveratrol Esters of Organic Acids

### Carboxylic Acid Esters of Resveratrol

The group of resveratrol derivatives to be used in the present invention is esters of resveratrol and aliphatic or aromatic carboxylic acids, in which one or more of X, Y, and Z is a -C(O)-Ri group, wherein Ri is selected from the group consisting of linear, branched, saturated or unsaturated, or cyclic C1-C40 alkyl, substituted C1-C40 alkyl, C1-C40 alkenyl, substituted C1-C40 alkenyl, C1-C40 alkynyl, substituted C1-C40 alkynyl, aryl, C1-C40 aryl, and C1-C40 substituted aryl. In one preferred embodiment, the R group is a straight or branched chain fatty, or C₆₋₃₀, saturated or unsaturated alkyl group. The substituents may be selected from C₁₋₄₀ straight or branched chain, saturated or unsaturated alkyl, halogen (such as fluoro), hydrogen, alkoxy, hydroxyl, and the like.

Exemplary carboxylic acids that can be used to form ester of resveratrol includesalicylic acid,. The designation "C" followed by a number indicates the number of carbon atoms in the alkyl chain. Carboxylic acid esters of resveratrol for practice of the present invention:
are the following:
3-salicylate-5,4'-dihydroxystilbene;
5-salicylate-3,4'-dihydroxystilbene;
4'-salicylate-3,5-dihydroxystilbene;
3,5-disalicylate-4'-hydroxystilbene;
3,4 '-disalicylate-5-hydroxystilbene;
4',5-disalicylate-3-hydroxystilbene;
3,5,4'-trisalicylate stilbene;

### EMBODIMENTS OF THE INVENTION

The invention has a number of embodiments which will be further described herein. Each embodiment provides for compositions containing certain essential ingredients, but which compositions may contain any one or more of the other ingredients listed as essential or non-essential for the other embodiments and in the same percentage ranges. By way of example only, the composition of Embodiment I is directed to an emulsion cosmetic composition comprising at least one resveratrol derivative as described herein and in the amounts specified, an aqueous phase, and oil phase, and at least one oil phase structuring agent. While the composition of Embodiment I contains these components as essential ingredients, it may contain one or more of any of the other ingredients listed as essential or non-essential in the compositions of the other embodiments and in the percentage ranges specified.

### Embodiment I

Embodiment I of the invention is directed to an emulsion cosmetic composition comprising at least one resveratrol derivative as described above and in the same percentage ranges, an aqueous phase, and an oil phase containing at least one oil phase structuring agent. The aqueous phase may be present ranging from about 0.1-99%, preferably from about 5-85%, more preferably from about 7-75% by weight of the total composition. The oil phase structuring agent contributes to an aesthetically pleasing emulsion that has a suitable texture and viscosity. In addition, the oil phase structuring agent contributes to the feel of the composition when applied, and makes it an appropriate consistency for storage in standard cosmetic containers such as tubes, jars, and the like.

### A. Oil Phase Ingredients

Suitable oils include silicones, esters, vegetable oils, synthetic oils, including but not limited to those set forth herein. Suggested ranges are from about 1-95%, preferably from about 5-85%, more preferably from about 7-65% by weight of the total composition.

The oils may be volatile, near volatile, or nonvolatile, and are in the form of a pourable liquid at room temperature. The term "volatile" means that the oil has a measurable vapor pressure, or a vapor pressure of at least about 2 mm. of mercury at 20° C. The term "near volatile" means that the oil has a vapor pressure ranging from about 1 to 2 mm. of mercury at 20° C. The term "nonvolatile" means that the oil has a vapor pressure of less than about 1 mm. of mercury at 20° C.

### 1. Volatile or Near Volatile Oils

### a. Volatile or Near Volatile Silicones

Suitable volatile or near volatile oils generally have a viscosity ranging from about 0.5 to 5 centistokes 25° C. and include linear silicones, cyclic silicones, paraffinic hydrocarbons, or mixtures thereof.
Cyclic silicones are of the general formula: 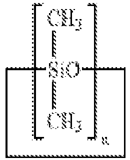 where n=3-6.

Linear volatile or near volatile silicones in accordance with the invention have the general formula:

(CH₃)₃Si-O-[Si(CH₃)₂-O]*ₙ*-Si(CH₃)₃

where n=0, 1, 2, 3, 4, 5, 6, 7, or 8; preferably 0, 1, 2, 3, or 4.

Linear and cyclic volatile or near volatile silicones are available from various commercial sources including Dow Corning Corporation and General Electric. The Dow Corning volatile silicones are sold under the tradenames Dow Corning 244, 245, 344, and 200 fluids. These fluids comprise octamethylcyclotetrasiloxane, decamethylcyclopentasiloxane, dodecamethylcyclohexasiloxane and the like. Also suitable are linear volatile silicones such as hexamethyldisiloxane (viscosity 0.65 centistokes (abbreviated cst)), octamethyltrisiloxane (1.0 cst), decamethyltetrasiloxane (1.5 cst), dodecamethylpentasiloxane (2 cst) and mixtures thereof.

Also suitable are branched volatile or near volatile silicones including methyl trimethicone which is a branched volatile silicone having the general formula:

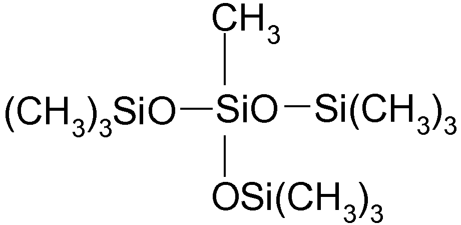

Methyl trimethicone may be purchased from Shin-Etsu Silicones under the tradename TMF- 1.5, having a viscosity of 1.5 centistokes at 25° C.

### b. Volatile or Near Volatile Paraffinic Hydrocarbons

Also suitable as the volatile or near volatile oils are various straight or branched chain paraffinic hydrocarbons having 5, 6, 7, 8, 9, 10, 11, 12, 13, 14, 15, 16, 17, 18, 19, or 20 carbon atoms, more preferably 8 to 16 carbon atoms. Suitable hydrocarbons include pentane, hexane, heptane, decane, dodecane, tetradecane, tridecane, and Cg-2o isoparaffins as disclosed in U.S. Pat. Nos. 3,439,088 and 3,818,105.

Preferred paraffinic hydrocarbons have a molecular weight of 70-225, preferably 160 to 190 and a boiling point range of 30 to 320, preferably 60 to 260° C, and a viscosity of less than about 10 cst. at 25° C. Such paraffinic hydrocarbons are available from EXXON under the ISOPARS trademark, and from the Permethyl Corporation. Suitable C₁₂ isoparaffins are manufactured by Permethyl Corporation under the tradename Permethyl 99A. Various C₁₆ isoparaffins commercially available, such as isohexadecane (having the tradename Permethyl R), are also suitable.

### 2. Non-Volatile Oils

A variety of nonvolatile oils are also suitable for use in the cosmetic compositions of the invention. The nonvolatile oils generally have a viscosity of greater than about 5 to 10 centistokes at 25° C., and may range in viscosity up to about 1,000,000 centipoise at 25° C. Examples of nonvolatile oils include, but are not limited to:

### a. Esters

Suitable esters are mono-, di-, and triesters. The composition may comprise one or more esters selected from the group, or mixtures thereof.

### (i) Monoesters

Monoesters are defined as esters formed by the reaction of a monocarboxylic acid having the formula R-COOH, wherein R is a straight or branched chain saturated or unsaturated alkyl having 2 to 45 carbon atoms, or phenyl; and an alcohol having the formula R-OH wherein R is a straight or branched chain saturated or unsaturated alkyl having 2-30 carbon atoms, or phenyl. Both the alcohol and the acid may be substituted with one or more hydroxyl groups. Either one or both of the acid or alcohol may be a "fatty" acid or alcohol, and may have from about 6 to 30 carbon atoms, more preferably 12, 14, 16, 18, or 22 carbon atoms in straight or branched chain, saturated or unsaturated form. Examples of monoester oils that may be used in the compositions of the invention include hexyl laurate, butyl isostearate, hexadecyl isostearate, cetyl palmitate, isostearyl neopentanoate, stearyl heptanoate, isostearyl isononanoate, steary lactate, stearyl octanoate, stearyl stearate, isononyl isononanoate, and so on.

### (ii). Diesters

Suitable diesters are the reaction product of a dicarboxylic acid and an aliphatic or aromatic alcohol or an aliphatic or aromatic alcohol having at least two substituted hydroxyl groups and a monocarboxylic acid. The dicarboxylic acid may contain from 2 to 30 carbon atoms, and may be in the straight or branched chain, saturated or unsaturated form. The dicarboxylic acid may be substituted with one or more hydroxyl groups. The aliphatic or aromatic alcohol may also contain 2 to 30 carbon atoms, and may be in the straight or branched chain, saturated, or unsaturated form. Preferably, one or more of the acid or alcohol is a fatty acid or alcohol, i.e. contains 12-22 carbon atoms. The dicarboxylic acid may also be an alpha hydroxy acid. The ester may be in the dimer or trimer form. Examples of diester oils that may be used in the compositions of the invention include diisotearyl malate, neopentyl glycol dioctanoate, dibutyl sebacate, dicetearyl dimer dilinoleate, dicetyl adipate, diisocetyl adipate, diisononyl adipate, diisostearyl dimer dilinoleate, diisostearyl fumarate, diisostearyl malate, dioctyl malate, and so on.

### (iii). Triesters

Suitable triesters comprise the reaction product of a tricarboxylic acid and an aliphatic or aromatic alcohol or alternatively the reaction product of an aliphatic or aromatic alcohol having three or more substituted hydroxyl groups with a monocarboxylic acid. As with the mono- and diesters mentioned above, the acid and alcohol contain 2 to 30 carbon atoms, and may be saturated or unsaturated, straight or branched chain, and may be substituted with one or more hydroxyl groups. Preferably, one or more of the acid or alcohol is a fatty acid or alcohol containing 12 to 22 carbon atoms. Examples of triesters include esters of arachidonic, citric, or behenic acids, such as triarachidin, tributyl citrate, triisostearyl citrate, tri C₁₂-₁₃ alkyl citrate, tricaprylin, tricaprylyl citrate, tridecyl behenate, trioctyldodecyl citrate, tridecyl behenate; or tridecyl cocoate, tridecyl isononanoate, and so on.

Esters suitable for use in the composition are further described on pages 1670-1676 of the C.T.F.A. Cosmetic Ingredient Dictionary and Handbook, Eighth Edition, 2000.

### b. Hydrocarbon Oils

It may be desirable to incorporate one or more nonvolatile hydrocarbon oils into the composition. Suitable nonvolatile hydrocarbon oils include paraffinic hydrocarbons and olefins, preferably those having greater than about 20 carbon atoms. Examples of such hydrocarbon oils include C24-28 olefins, C30-45 olefins, C20-40 isoparaffins, hydrogenated polyisobutene, polyisobutene, polydecene, hydrogenated polydecene, mineral oil, pentahydrosqualene, squalene, squalane, and mixtures thereof. In one preferred embodiment such hydrocarbons have a molecular weight ranging from about 300 to 1000 Daltons.

### c. Glyceryl Esters of Fatty Acids

Synthetic or naturally occurring glyceryl esters of fatty acids, or triglycerides, are also suitable for use in the compositions. Both vegetable and animal sources may be used. Examples of such oils include castor oil, lanolin oil, C10-18 triglycerides, caprylic/capric/triglycerides, sweet almond oil, apricot kernel oil, sesame oil, camelina sativa oil, tamanu seed oil, coconut oil, corn oil, cottonseed oil, linseed oil, ink oil, olive oil, palm oil, illipe butter, rapeseed oil, soybean oil, grapeseed oil, sunflower seed oil, walnut oil, and the like.

Also suitable are synthetic or semi-synthetic glyceryl esters, such as fatty acid mono-, di-, and triglycerides which are natural fats or oils that have been modified, for example, mono-, di- or triesters of polyols such as glycerin. In an example, a fatty (C₁₂₋₂₂) carboxylic acid is reacted with one or more repeating glyceryl groups, glyceryl stearate, diglyceryl diiosostearate, polyglyceryl-3 isostearate, polyglyceryl-4 isostearate, polyglyceryl-6 ricinoleate, glyceryl dioleate, glyceryl diisotearate, glyceryl tetraisostearate, glyceryl trioctanoate, diglyceryl distearate, glyceryl linoleate, glyceryl myristate, glyceryl isostearate, PEG castor oils, PEG glyceryl oleates, PEG glyceryl stearates, PEG glyceryl tallowates, and so on.

### d. Nonvolatile Silicones

Nonvolatile silicone oils, both water soluble and water insoluble, are also suitable for use in the composition. Such silicones preferably have a viscosity ranging from about 10 to 800,000 cst, preferably 20 to 200,000 cst at 25° C. Suitable water insoluble silicones include amine functional silicones such as amodimethicone; phenyl substituted silicones such as bisphenylhexamethicone, trimethylsiloxyphenyl dimethicone, phenyl trimethicone, or polyphenylmethylsiloxane; dimethicone, dimethicone substituted with C₂₋₃₀ alkyl groups such cetyl dimethicone.

Nonvolatile silicones may have the following general formula: 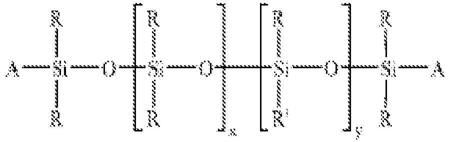 wherein R and R' are each independently C₁₋₃₀ straight or branched chain, saturated or unsaturated alkyl, phenyl or aryl, trialkylsiloxy, and x and y are each independently 0-1,000,000; with the proviso that there is at least one of either x or y, and A is alkyl siloxy endcap unit. Preferred is where A is a methyl siloxy endcap unit; in particular trimethylsiloxy, and R and R' are each independently a C₁₋₃₀ straight or branched chain alkyl, phenyl, or trimethylsiloxy, more preferably a C₁-₂₂ alkyl, phenyl, or trimethylsiloxy, most preferably methyl, phenyl, or trimethylsiloxy, and resulting silicone is dimethicone, phenyl dimethicone, diphenyl dimethicone, phenyl trimethicone, or trimethylsiloxyphenyl dimethicone. Other examples include alkyl dimethicones such as cetyl dimethicone, and the like wherein at least one R is a fatty alkyl (C12, C14, C16, C18, C2o, or C22), and the other R is methyl, and A is a trimethylsiloxy endcap unit, provided such alkyl dimethicone is a pourable liquid at room temperature. Phenyl trimethicone can be purchased from Dow Corning Corporation under the tradename 556 Fluid. Trimethylsiloxyphenyl dimethicone can be purchased from Wacker-Chemie under the tradename PDM-1000. Cetyl dimethicone, also referred to as a liquid silicone wax, may be purchased from Dow Corning as Fluid 2502, or from DeGussa Care & Surface Specialties under the trade names Abil Wax 9801, or 9814.

### e. Fluorinated Oils

Various types of fluorinated oils may also be suitable for use in the compositions including but not limited to fluorinated silicones, fluorinated esters, or perfluropoly ethers. Particularly suitable are fluorosilicones such as trimethylsilyl endcapped fluorosilicone oil, polytrifluoropropylmethylsiloxanes, and similar silicones such as those disclosed in U.S. Pat. No. 5,118,496. Perfluoropolyethers include those disclosed in U.S. Pat. Nos. 5,183,589, 4,803,067, 5,183,588, which are commercially available from Montefluos under the trademark Fomblin.

### B. Oil Phase Structuring Agents

The invention of Embodiment I will comprise at least one oil phase structuring agent.

A variety of oil phase structuring agents may be present. The term "oil phase structuring agent" means an ingredient or combination of ingredients, soluble or dispersible in the oil phase, which will increase the viscosity, or structure, the oil phase. The oil phase structuring agent is compatible with the resveratrol derivative and the rest of the formulation ingredients. The term "compatible" means that the oil phase structuring agent and resveratrol derivative are capable of being formulated into a cosmetic product that is generally stable. The structuring agent may be present in an amount sufficient to provide a liquid composition with increased viscosity, a semi-solid, or in some cases a solid composition that may be self-supporting. The structuring agent itself may be present in the liquid, semi-solid, or solid form. Suggested ranges of structuring agent are from about 0.01 to 70%, preferably from about 0.05 to 50%, more preferably from about 0.1-35% by weight of the total composition. Suitable oil phase structuring agents include those that are silicone based or organic based. They may be polymers or non-polymers, synthetic, natural, or a combination of both.

### 1. Silicone Structuring Agents

A variety of oil phase structuring agents may be silicone based, such as silicone elastomers, silicone gums, silicone waxes, linear silicones having a degree of polymerization that provides the silicone with a degree of viscosity such that when incorporated into the cosmetic composition it is capable of increasing the viscosity of the oil phase. Examples of silicone structuring agents include, but are not limited to:

### a. Silicone Elastomers

Silicone elastomers suitable for use in the compositions of the invention include those that are formed by addition reaction-curing, by reacting an SiH-containing diorganosiloxane and an organopolysiloxane having terminal olefinic unsaturation, or an alpha-omega diene hydrocarbon, in the presence of a platinum metal catalyst. Such elastomers may also be formed by other reaction methods such as condensation-curing organopolysiloxane compositions in the presence of an organotin compound via a dehydrogenation reaction between hydroxyl-terminated diorganopolysiloxane and SiH-containing diorganopolysiloxane or alpha omega diene; or by condensation-curing organopolysiloxane compositions in the presence of an organotin compound or a titanate ester using a condensation reaction between an hydroxyl-terminated diorganopolysiloxane and a hydrolysable organosiloxane; peroxide-curing organopolysiloxane compositions which thermally cure in the presence of an organoperoxide catalyst.

One type of elastomer that may be suitable is prepared by addition reaction-curing an organopolysiloxane having at least 2 lower alkenyl groups in each molecule or an alpha-omega diene; and an organopolysiloxane having at least 2 silicon-bonded hydrogen atoms in each molecule; and a platinum-type catalyst. While the lower alkenyl groups such as vinyl, can be present at any position in the molecule, terminal olefinic unsaturation on one or both molecular terminals is preferred. The molecular structure of this component may be straight chain, branched straight chain, cyclic, or network. These organopolysiloxanes are exemplified by methylvinylsiloxanes, methylvinylsiloxane-dimethylsiloxane copolymers, dimethylvinylsiloxy-terminated dimethylpolysiloxanes, dimethylvinylsiloxy-terminated dimethylsiloxane-methylphenylsiloxane copolymers, dimethylvinylsiloxy-terminated dimethylsiloxane-diphenylsiloxane-methylvinylsiloxane copolymers, trimethylsiloxy-terminated dimethylsiloxane-methylvinylsiloxane copolymers, trimethylsiloxy-terminated dimethylsiloxane-methylphenylsiloxane-methylvinylsiloxane copolymers, dimethylvinylsiloxy-terminated methyl(3,3,3-trifluoropropyl) polysiloxanes, and dimethylvinylsiloxy-terminated dimethylsiloxane-methyl(3,3,-trifluoropropyl)siloxane copolymers, decadiene, octadiene, heptadiene, hexadiene, pentadiene, or tetradiene, or tridiene.

Curing proceeds by the addition reaction of the silicon-bonded hydrogen atoms in the dimethyl methylhydrogen siloxane, with the siloxane or alpha-omega diene under catalysis using the catalyst mentioned herein. To form a highly crosslinked structure, the methyl hydrogen siloxane must contain at least 2 silicon-bonded hydrogen atoms in each molecule in order to optimize function as a crosslinker.

The catalyst used in the addition reaction of silicon-bonded hydrogen atoms and alkenyl groups, and is concretely exemplified by chloroplatinic acid, possibly dissolved in an alcohol or ketone and this solution optionally aged, chloroplatinic acid-olefin complexes, chloroplatinic acid-alkenylsiloxane complexes, chloroplatinic acid-diketone complexes, platinum black, and carrier-supported platinum.

Examples of suitable silicone elastomers for use in the compositions of the invention may be in the powder form, or dispersed or solubilized in solvents such as volatile or non-volatile silicones, or silicone compatible vehicles such as paraffinic hydrocarbons or esters. Examples of silicone elastomer powders include vinyl dimethicone/methicone silesquioxane crosspolymers like Shin-Etsu's KSP-100, KSP-101, KSP- 102, KSP- 103, KSP- 104, KSP-105, hybrid silicone powders that contain a fluoroalkyl group like Shin-Etsu's KSP-200 which is a fluoro-silicone elastomer, and hybrid silicone powders that contain a phenyl group such as Shin-Etsu's KSP-300, which is a phenyl substituted silicone elastomer; and Dow Coming's DC 9506. Examples of silicone elastomer powders dispersed in a silicone compatible vehicle include dimethicone/vinyl dimethicone crosspolymers supplied by a variety of suppliers including Dow Corning Corporation under the tradenames 9040 or 9041, GE Silicones under the tradename SFE 839, or Shin-Etsu Silicones under the tradenames KSG-15, 16, 18. KSG-15 has the CTFA name cyclopentasiloxane/dimethicone/vinyl dimethicone crosspolymer. KSG- 18 has the INCI name phenyl trimethicone/dimethicone/phenyl vinyl dimethicone crossoplymer. Silicone elastomers may also be purchased from Grant Industries under the Gransil trademark. Also suitable are silicone elastomers having long chain alkyl substitutions such as lauryl dimethicone/vinyl dimethicone crosspolymers supplied by Shin Etsu under the tradenames KSG-31, KSG-32, KSG-41, KSG-42, KSG-43, and KSG-44. Cross-linked organopolysiloxane elastomers useful in the present invention and processes for making them are further described in U.S. Pat. No. 4,970,252 to Sakuta et al, issued Nov. 13, 1990; U.S. Pat. No. 5,760,116 to Kilgour et al., issued Jun. 2, 1998; U.S. Pat. No. 5,654,362 to Schulz, Jr. et al. issued Aug. 5, 1997; and Japanese Patent Application JP 61-18708, assigned to Pola Kasei Kogyo KK,.

### b. Silicone Gums

Also suitable for use as an oil phase structuring agent are one or more silicone gums. The term "gum" means a silicone polymer having a degree of polymerization sufficient to provide a silicone having a gum-like texture. In certain cases the silicone polymer forming the gum may be crosslinked. The silicone gum typically has a viscosity ranging from about 500,000 to 100 million cst at 25° C., preferably from about 600,000 to 20 million, more preferably from about 600,000 to 12 million cst. All ranges mentioned herein include all subranges, e.g. 550,000; 925,000; 3.5 million.

The silicone gums that are used in the compositions include, but are not limited to, those of the general formula wherein: 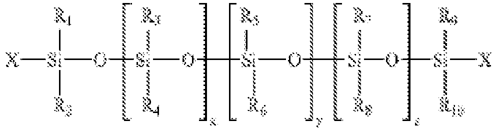 R₁ to R₉ are each independently an alkyl having 1 to 30 carbon atoms, aryl, or aralkyl; and X is OH or a C₁-₃₀ alkyl, or vinyl; and wherein x, y, or z may be zero with the proviso that no more than two of x, y, or z are zero at any one time, and further that x, y, and z are such that the silicone gum has a viscosity of at least about 500,000 cst, ranging up to about 100 million centistokes at 25° C. Preferred is where R is methyl or OH.

Such silicone gums may be purchased in pure form from a variety of silicone manufacturers including Wacker-Chemie or Dow Corning, and the like. Such silicone gums include those sold by Wacker-Belsil under the trade names CM3092, Wacker-Belsil 1000, or Wacker-Belsil DM 3096. A silicone gum where X is OH, also referred to as dimethiconol, is available from Dow Corning Corporation under the trade name 1401. The silicone gum may also be purchased in the form of a solution or dispersion in a silicone compatible vehicle such as volatile or nonvolatile silicone. An example of such a mixture may be purchased from Barnet Silicones under the HL-88 tradename, having the INCI name dimethicone.

### c. Silicone Waxes

Another type of oily phase structuring agent includes silicone waxes that are typically referred to as alkyl silicone waxes which are semi-solids or solids at room temperature. The term "alkyl silicone wax" means a polydimethylsiloxane having a substituted long chain alkyl (such as C16 to 30) that confers a semi-solid or solid property to the siloxane. Examples of such silicone waxes include stearyl dimethicone, which may be purchased from DeGussa Care & Surface Specialties under the tradename Abil Wax 9800 or from Dow Corning under the tradename 2503. Another example is bis-stearyl dimethicone, which may be purchased from Gransil Industries under the tradename Gransil A-18, or behenyl dimethicone, behenoxy dimethicone.

### 2. Other Structuring Agents

### a. Polyamides or Silicone Polyamides

Also suitable as oil phase structuring agents are various types of polymeric compounds such as polyamides or silicone polyamides.

The term silicone polyamide means a polymer comprised of silicone monomers and monomers containing amide groups as further described herein. The silicone polyamide preferably comprises moieties of the general formula: 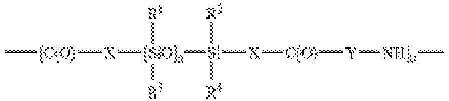 X is a linear or branched alkylene having from about 1-30 carbon atoms; R₁, R₂, R₃, and R₄ are each independently C₁₋₃₀ straight or branched chain alkyl which may be substituted with one or more hydroxyl or halogen groups; phenyl which may be substituted with one or more C₁₋₃₀ alkyl groups, halogen, hydroxyl, or alkoxy groups; or a siloxane chain having the general formula: 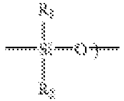 and Y is:
(a) a linear or branched alkylene having from about 1-40 carbon atoms which may be substituted with (i) one or more amide groups having the general formula R₁CONR₁, or (ii) C₅₋₆ cyclic ring, or (iii) phenylene which may be substituted with one or more C₁₋₁₀ alkyl groups, or (iv) hydroxy, or (v) C₃₋₈ cycloalkane, or (vi) C₁₋₂₀ alkyl which may be substituted with one or more hydroxy groups, or (vii) C₁₋₁₀ alkyl amines; or
(b) TR₅R₆R₇
wherein R₅, R₆, and R₇, are each independently a C₁₋₁₀ linear or branched alkylenes, and T is CR₈ wherein R₈ is hydrogen, a trivalent atom N, P, or Al, or a C₁₋₃₀ straight or branched chain alkyl which may be substituted with one or more hydroxyl or halogen groups; phenyl which may be substituted with one or more C₁₋₃₀ alkyl groups, halogen, hydroxyl, or alkoxy groups; or a siloxane chain having the general formula:

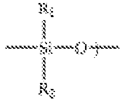

Preferred is where R₁, R₂, R₃, and R₄ are C₁₋₁₀, preferably methyl; and X and Y is a linear or branched alkylene. Preferred are silicone polyamides having the general formula 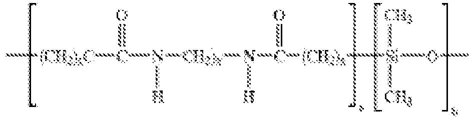 wherein a and b are each independently sufficient to provide a silicone polyamide polymer having a melting point ranging from about 60 to 120° C., and a molecular weight ranging from about 40,000 to 500,000 Daltons. One type of silicone polyamide that may be used in the compositions of the invention may be purchased from Dow Corning Corporation under the tradename Dow Corning 2-8178 gellant which has the CTFA name nylon-611/dimethicone copolymer which is sold in a composition containing PPG-3 myristyl ether. Also suitable are polyamides such as those purchased from Arizona Chemical under the tradenames Uniclear and Sylvaclear. Such polyamides may be ester terminated or amide terminated. Examples of ester terminated polyamides include, but are not limited to those having the general formula: 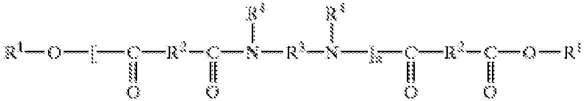 wherein n denotes a number of amide units such that the number of ester groups ranges from about 10% to 50% of the total number of ester and amide groups; each R₁ is independently an alkyl or alkenyl group containing at least 4 carbon atoms; each R₂ is independently a C₄₋₄₂ hydrocarbon group, with the proviso that at least 50% of the R₂ groups are a C30-42 hydrocarbon; each R₃ is independently an organic group containing at least 2 carbon atoms, hydrogen atoms and optionally one or more oxygen or nitrogen atoms; and each R₄ is independently a hydrogen atom, a C₁₋₁₀ alkyl group or a direct bond to R₃ or to another R₄, such that the nitrogen atom to which R₃ and R₄ are both attached forms part of a heterocyclic structure defined by R₄-N-R₃, with at least 50% of the groups R₄ representing a hydrogen atom.

General examples of ester and amide terminated polyamides that may be used as oil phase gelling agents include those sold by Arizona Chemical under the tradenames Sylvaclear A200V or A2614V, both having the CTFA name ethylenediamine/hydrogenated dimer dilinoleate copolymer/bis-di-C₁₄₋₁₈ alkyl amide; Sylvaclear AF1900V; Sylvaclear C75V having the CTFA name bis-stearyl ethylenediamine/neopentyl glycol/stearyl hydrogenated dimer dilinoleate copolymer; Sylvaclear PA1200V having the CTFA name Polyamide-3; Sylvaclear PE400V; Sylvaclear WF1500V; or Uniclear, such as Uniclear 100VG having the INCI name ethylenediamine/stearyl dimer dilinoleate copolymer; or ethylenediamine/stearyl dimer ditallate copolymer. Other examples of suitable polyamides include those sold by Henkel under the Versamid trademark (such as Versamid 930, 744, 1655), or by Olin Mathieson Chemical Corp. under the brand name Onamid S or Onamid C.

### b. Natural or Synthetic Organic Waxes

Also suitable as the oil phase structuring agent may be one or more natural or synthetic waxes such as animal, vegetable, or mineral waxes. Preferably such waxes will have a higher melting point such as from about 60 to 150° C., more preferably from about 65 to 100° C. Examples of such waxes include waxes made by Fischer-Tropsch synthesis, such as polyethylene or synthetic wax; or various vegetable waxes such as bayberry, candelilla, ozokerite, acacia, beeswax, ceresin, cetyl esters, flower wax, citrus wax, carnauba wax, jojoba wax, japan wax, polyethylene, microcrystalline, rice bran, lanolin wax, mink, montan, bayberry, ouricury, ozokerite, palm kernel wax, paraffin, avocado wax, apple wax, shellac wax, clary wax, spent grain wax, grape wax, and polyalkylene glycol derivatives thereof such as PEG6-20 beeswax, or PEG-12 carnauba wax; or fatty acids or fatty alcohols, including esters thereof, such as hydroxystearic acids (for example 12-hydroxy stearic acid), tristearin, tribehenin, and so on.

### c. Montmorillonite Minerals

One type of structuring agent that may be used in the composition comprises natural or synthetic montmorillonite minerals such as hectorite, bentonite, and quaternized derivatives thereof, which are obtained by reacting the minerals with a quaternary ammonium compound, such as stearalkonium bentonite, hectorites, quaternized hectorites such as Quaternium-18 hectorite, attapulgite, carbonates, bentones, and the like.

### d. Silicas and Silicates

Another type of structuring agent that may be used in the compositions are silicas, silicates, silica silylate, and alkali metal or alkaline earth metal derivatives thereof. These silicas and silicates are generally found in the particulate form and include silica, silica silylate, magnesium aluminum silicate, and the like.

### C. Other Ingredients

The composition of Embodiment I may additionally contain a variety of other ingredients as further set forth herein and in the same percentage ranges.

### A.

In addition, the compositions of Embodiment I may optionally contain one or more silicone surfactants and in the percentage ranges set forth herein.

### A. Dimethicone Copolyols or Alkyl Dimethicone Copolyols

One type of silicone surfactant that may be used is generally referred to as dimethicone copolyol or alkyl dimethicone copolyol. This surfactant is either a water-in-oil or oil-in- water surfactant having an Hydrophile/Lipophile Balance (HLB) ranging from about 2 to 18. Preferably the silicone surfactant is a nonionic surfactant having an HLB ranging from about 2 to 12, preferably about 2 to 10, most preferably about 4 to 6. The term "hydrophilic radical" means a radical that, when substituted onto the organosiloxane polymer backbone, confers hydrophilic properties to the substituted portion of the polymer. Examples of radicals that will confer hydrophilicity are hydroxy-polyethyleneoxy, hydroxyl, carboxylates, and mixtures thereof. The term "lipophilic radical" means an organic radical that, when substituted onto the organosiloxane polymer backbone, confers lipophilic properties to the substituted portion of the polymer. Examples of organic radicals that will confer lipophilicity are Ci_4o straight or branched chain alkyl, fluoro, aryl, aryloxy, Ci_4o hydrocarbyl acyl, hydroxy-polypropyleneoxy, or mixtures thereof.

One type of suitable silicone surfactant has the general formula: 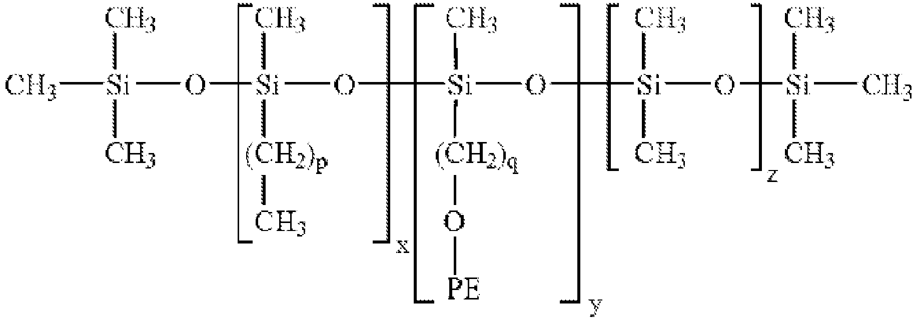 wherein p and q are from 0 to 40 (the range including all numbers between and subranges such as 2, 3, 4, 13, 14, 15, 16, 17, 18, etc.), and PE is (-C₂H₄O)ₐ-(-C₃H₆O)_{b}-H, wherein a is from 0 to 25, b is from 0 to 25, with the proviso that a and b cannot both be 0 simultaneously, wherein x, y and z are each independently ranging from 0 to 1 million, with the proviso that they cannot all be 0 simultaneously. In one preferred embodiment, x, y, z, a, and b are such that the molecular weight of the polymer ranges from about 5,000 to about 500,000, more preferably from about 10,000 to 100,000, and is most preferably approximately about 50,000 and the polymer is generically referred to as dimethicone copolyol.

One type of silicone surfactant is wherein p is such that the long chain alkyl is cetyl or lauryl, and the surfactant is called, generically, cetyl dimethicone copolyol or lauryl dimethicone copolyol respectively.

In some cases the number of repeating ethylene oxide or propylene oxide units in the polymer are also specified, such as a dimethicone copolyol that is also referred to as PEG-15/PPG-10 dimethicone, which refers to a dimethicone having substituents containing 15 ethylene glycol units and 10 propylene glycol units on the siloxane backbone. It is also possible for one or more of the methyl groups in the above general structure to be substituted with a longer chain alkyl (e.g. ethyl, propyl, butyl, etc.) or an ether such as methyl ether, ethyl ether, propyl ether, butyl ether, and the like.

Examples of silicone surfactants are those sold by Dow Corning under the tradename Dow Corning 3225C Formulation Aid having the CTFA name cyclotetrasiloxane (and) cyclopentasiloxane (and) PEG/PPG-18 dimethicone; or 5225C Formulation Aid, having the CTFA name cyclopentasiloxane (and) PEG/PPG-18/18 dimethicone; or Dow Coming 190 Surfactant having the CTFA name PEG/PPG-18/18 dimethicone; or Dow Corning 193 Fluid, Dow Corning 5200 having the CTFA name lauryl PEG/PPG-18/18 methicone; or Abil EM 90 having the CTFA name cetyl PEG/PPG-14/14 dimethicone sold by Goldschmidt; or Abil EM 97 having the CTFA name bis-cetyl PEG/PPG-14/14 dimethicone sold by Goldschmidt; or Abil WE 09 having the CTFA name cetyl PEG/PPG-10/1 dimethicone in a mixture also containing polyglyceryl-4 isostearate and hexyl laurate; or KF-6011 sold by Shin-Etsu Silicones having the CTFA name PEG-11 methyl ether dimethicone; KF-6012 sold by Shin-Etsu Silicones having the CTFA name PEG/PPG-20/22 butyl ether dimethicone; or KF-6013 sold by Shin-Etsu Silicones having the CTFA name PEG-9 dimethicone; or KF-6015 sold by Shin-Etsu Silicones having the CTFA name PEG-3 dimethicone; or KF-6016 sold by Shin-Etsu Silicones having the CTFA name PEG-9 methyl ether dimethicone; or KF-6017 sold by Shin-Etsu Silicones having the CTFA name PEG-10 dimethicone; or KF-6038 sold by Shin-Etsu Silicones having the CTFA name lauryl PEG-9 polydimethylsiloxyethyl dimethicone.

### B. Crosslinked Silicone Surfactants

Also suitable are various types of crosslinked silicone surfactants are referred to as emulsifying elastomers. They are typically prepared as set forth above with respect to the section "silicone elastomers" except that the silicone elastomers will contain at least one hydrophilic moiety such as polyoxyalkylenated groups. Typically these polyoxyalkylenated silicone elastomers are crosslinked organopolysiloxanes that may be obtained by a crosslinking addition reaction of diorganopolysiloxane comprising at least one hydrogen bonded to silicon and of a polyoxyalkylene comprising at least two ethylenically unsaturated groups. In at least one embodiment, the polyoxyalkylenated crosslinked organo-polysiloxanes are obtained by a crosslinking addition reaction of a diorganopolysiloxane comprising at least two hydrogens each bonded to a silicon, and a polyoxyalkylene comprising at least two ethylenically unsaturated groups, optionally in the presence of a platinum catalyst, as described, for example, in U.S. Pat. No. 5,236,986 and U.S. Pat. No. 5,412,004, U.S. Pat. No. 5,837,793 and U.S. Pat. No. 5,811,487,.

Polyoxyalkylenated silicone elastomers that may be used in at least one embodiment of the invention include those sold by Shin-Etsu Silicones under the names KSG-21 , KSG-20, KSG-30, KSG-31, KSG-32, KSG-33; KSG-210 which is dimethicone/PEG-10/15 crosspolymer dispersed in dimethicone; KSG-310 which is PEG- 15 lauryl dimethicone crosspolymer; KSG-320 which is PEG- 15 lauryl dimethicone crosspolymer dispersed in isododecane; KSG-330 (the former dispersed in triethylhexanoin), KSG-340 which is a mixture of PEG-IO lauryl dimethicone crosspolymer and PEG- 15 lauryl dimethicone crosspolymer.

Also suitable are polyglycerolated silicone elastomers like those disclosed in PCT/WO 2004/024798,. Such elastomers include Shin-Etsu' s KSG series, such as KSG-710 which is dimethicone/polyglycerin-3 crosspolymer dispersed in dimethicone; or lauryl dimethicone/polyglycerin-3 crosspolymer dispersed in a variety of solvent such as isododecane, dimethicone, triethylhexanoin, sold under the Shin-Etsu tradenames KSG-810, KSG-820, KSG-830, or KSG-840. Also suitable are silicones sold by Dow Corning under the tradenames 9010 and DC9011

One preferred crosslinked silicone elastomer emulsifier is dimethicone/PEG-10/15 crosspolymer.

### VI. Other Ingredients

### A. Other Surfactants

The compositions of Embodiment I may contain one or more surfactants. The surfactants will aid in the formation of stable emulsions of either the water-in-oil or oil-in-water form. If present, such surfactants may range from about 0.001 to 30%, preferably from about 0.005 to 25%, more preferably from about 0.1 to 20% by weight of the total composition. The silicone surfactants are optional components of the compositions in Embodiment I. Other surfactants that may be present in the Embodiment I compositions include but are not limited to:

### 1. Organic Nonionic Surfactants

Suitable nonionic surfactants include alkoxylated alcohols, or ethers, formed by the reaction of an alcohol with an alkylene oxide, usually ethylene or propylene oxide. Preferably the alcohol is either a fatty alcohol having 6 to 30 carbon atoms Examples of such ingredients include Steareth 2-100, which is formed by the reaction of stearyl alcohol and ethylene oxide and the number of ethylene oxide units ranges from 2 to 100; Beheneth 5-30 which is formed by the reaction of behenyl alcohol and ethylene oxide where the number of repeating ethylene oxide units is 5 to 30; Ceteareth 2-100, formed by the reaction of a mixture of cetyl and stearyl alcohol with ethylene oxide, where the number of repeating ethylene oxide units in the molecule is 2 to 100; Ceteth 1-45 which is formed by the reaction of cetyl alcohol and ethylene oxide, and the number of repeating ethylene oxide units is 1 to 45, and so on. Other alkoxylated alcohols are formed by the reaction of fatty acids and mono-, di- or polyhydric alcohols with an alkylene oxide. For example, the reaction products of C₆₋₃₀ fatty carboxylic acids and polyhydric alcohols which are monosaccharides such as glucose, galactose, methyl glucose, and the like, with an alkoxylated alcohol. Examples include polymeric alkylene glycols reacted with glyceryl fatty acid esters such as PEG glyceryl oleates, PEG glyceryl stearate; or PEG polyhydroxyalkanotes such as PEG dipolyhydroxystearate wherein the number of repeating ethylene glycol units ranges from 3 to 1000.

Also suitable as nonionic surfactants are formed by the reaction of a carboxylic acid with an alkylene oxide or with a polymeric ether. The resulting products have the general formula: where RCO is the carboxylic ester radical, X is hydrogen or lower alkyl, and n is the number of polymerized alkoxy groups. In the case of the diesters, the two RCO-groups do not need to be identical. Preferably, R is a C6-30 straight or branched chain, saturated or unsaturated alkyl, and n is from 1-100.

Monomeric, homopolymeric, or block copolymeric ethers are also suitable as nonionic surfactants. Typically, such ethers are formed by the polymerization of monomeric alkylene oxides, generally ethylene or propylene oxide. Such polymeric ethers have the following general formula: wherein R is H or lower alkyl and n is the number of repeating monomer units, and ranges from 1 to 500.

Other suitable nonionic surfactants include alkoxylated sorbitan and alkoxylated sorbitan derivatives. For example, alkoxylation, in particular ethoxylation of sorbitan provides polyalkoxylated sorbitan derivatives. Esterification of polyalkoxylated sorbitan provides sorbitan esters such as the polysorbates. For example, the polyalkyoxylated sorbitan can be esterified with C6-30, preferably C12-22 fatty acids. Examples of such ingredients include Polysorbates 20-85, sorbitan oleate, sorbitan sesquioleate, sorbitan palmitate, sorbitan sesquiisostearate, sorbitan stearate, and so on.

Certain types of amphoteric, zwitterionic, or cationic surfactants may also be used in the compositions. Descriptions of such surfactants are set forth in U.S. Pat. No. 5,843,193,.

### B. Humectants

It may also be desirable to include one or more humectants in the compositions of Embodiments I-V. If present, such humectants may range from about 0.001 to 25%, preferably from about 0.005 to 20%, more preferably from about 0.1 to 15% by weight of the total composition. Examples of suitable humectants include glycols, sugars, and the like. Suitable glycols are in monomeric or polymeric form and include polyethylene and polypropylene glycols such as PEG 4-200, which are polyethylene glycols having from 4 to 200 repeating ethylene oxide units; as well as Ci_6 alkylene glycols such as propylene glycol, butylene glycol, pentylene glycol, and the like. Suitable sugars, some of which are also polyhydric alcohols, are also suitable humectants. Examples of such sugars include glucose, fructose, honey, hydrogenated honey, inositol, maltose, mannitol, maltitol, sorbitol, sucrose, xylitol, xylose, and so on. Preferably, the humectants used in the composition of the invention are C₁₋₆, preferably C₂₋₄ alkylene glycols, most particularly butylene glycol.

### C. Botanical Extracts

It may be desirable to include one or more botanical extracts in the compositions of Embodiments I-V. If so, suggested ranges are from about 0.0001 to 10%, preferably about 0.0005 to 8%, more preferably about 0.001 to 5% by weight of the total composition. Suitable botanical extracts include extracts from plants (herbs, roots, flowers, fruits, seeds) such as flowers, fruits, vegetables, and so on, including yeast ferment extract, padica pavonica extract, thermus thermophilis ferment extract, camelina sativa seed oil, boswellia serrata extract, olive extract, aribodopsis thaliana extract, acacia dealbata extract, acer saccharinum (sugar maple), acidopholus, acorus, aesculus, agaricus, agave, agrimonia, algae, aloe, citrus, brassica, cinnamon, orange, apple, blueberry, cranberry, peach, pear, lemon, lime, pea, seaweed, caffeine, green tea, chamomile, willowbark, mulberry, poppy, and those set forth on pages 1646 through 1660 of the CTFA Cosmetic Ingredient Handbook, Eighth Edition, Volume 2. Further specific examples include, but are not limited to, Glycyrrhiza Glabra, Salix Nigra, Macrocycstis Pyrifera, Pyrus Malus, Saxifraga Sarmentosa, Vilis Vinifera, Morus Nigra, Scutellaria Baicalensis, Anthemis Nobilis, Salvia Sclarea, Rosmarinus Officianalis, Citrus Medica Limonum, Panax Ginseng, and mixtures thereof.

### D. Sunscreens

It may also be desirable to include one or more sunscreens in the compositions of Embodiments I-V. Such sunscreens include chemical UVA or UVB sunscreens or physical sunscreens in the particulate form.

### 1. UVA Chemical Sunscreens

If desired, the composition may comprise one or more UVA sunscreens. The term "UVA sunscreen" means a chemical compound that blocks UV radiation in the wavelength range of about 320 to 400 nm. Preferred UVA sunscreens are dibenzoylmethane compounds having the general formula 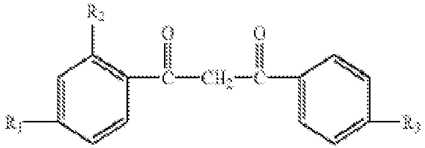 wherein R₁ is H, OR and NRR wherein each R is independently H, C₁₋₂₀ straight or branched chain alkyl; R₂ is H or OH; and R₃ is H, C₁₋₂₀ straight or branched chain alkyl.

Preferred is where R₁ is OR where R is a C₁₋₂₀ straight or branched alkyl, preferably methyl; R₂ is H; and R₃ is a C₁₋₂₀ straight or branched chain alkyl, more preferably, butyl.

Examples of suitable UVA sunscreen compounds of this general formula include 4-methyldibenzoylmethane, 2-methyldibenzoylmethane, 4-isopropyldibenzoylmethane, 4-tert-butyldibenzoylmethane, 2,4-dimethyldibenzoylmethane, 2,5-dimethyldibenzoylmethane, 4,4'diisopropylbenzoylmethane, 4-tert-butyl-4'-methoxydibenzoylmethane, 4,4'-diisopropylbenzoylmethane, 2-methyl-5-isopropyl-4'-methoxydibenzoymethane, 2-methyl-5-tert-butyl-4'-methoxydibenzoylmethane, and so on. Particularly preferred is 4-tert-butyl-4'-methoxydibenzoylmethane, also referred to as Avobenzone. Avobenzone is commercial available from Givaudan-Roure under the trademark Parsol 1789, and Merck & Co. under the tradename Eusolex 9020.

The composition may contain from about 0.001-20%, preferably 0.005-5%, more preferably about 0.005-3% by weight of the composition of UVA sunscreen. In the preferred embodiment of the invention the UVA sunscreen is Avobenzone, and it is present at not greater than about 3% by weight of the total composition.

### 2. UVB Chemical Sunscreens

The term "UVB sunscreen" means a compound that blocks UV radiation in the wavelength range of from about 290 to 320 nm. A variety of UVB chemical sunscreens exist including alpha-cyano-beta,beta-diphenyl acrylic acid esters as set forth in U.S. Pat. No. 3,215,724,. One particular example of an alpha-cyano-beta,beta-diphenyl acrylic acid ester is Octocrylene, which is 2-ethylhexyl 2- cyano-3,3-diphenylacrylate. In certain cases the composition may contain no more than about 110% by weight of the total composition of octocrylene. Suitable amounts range from about 0.001-10% by weight. Octocrylene may be purchased from BASF under the tradename Uvinul N-539.

Other suitable sunscreens include benzylidene camphor derivatives as set forth in U.S. Pat. No. 3,781,417. Such benzylidene camphor derivatives have the general formula: 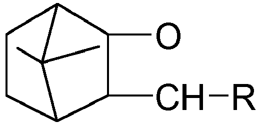 wherein R is p-tolyl or styryl, preferably styryl. Particularly preferred is 4-methylbenzylidene camphor, which is a lipid soluble UVB sunscreen compound sold under the tradename Eusolex 6300 by Merck.
Also suitable are cinnamate derivatives having the general formula: 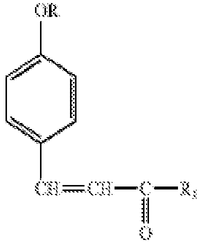 wherein R and R₁ are each independently a C₁₋₂₀ straight or branched chain alkyl. Preferred is where R is methyl and R₁ is a branched chain C₁₋₁₀, preferably C₈ alkyl. The preferred compound is ethylhexyl methoxycinnamate, also referred to as Octoxinate or octyl methoxycinnamate. The compound may be purchased from Givaudan Corporation under the tradename Parsol MCX, or BASF under the tradename Uvinul MC 80. Also suitable are mono-, di-, and triethanolamine derivatives of such methoxy cinnamates including diethanolamine methoxycinnamate. Cinoxate, the aromatic ether derivative of the above compound is also acceptable. If present, the Cinoxate should be found at no more than about 3% by weight of the total composition.

Also suitable as UVB screening agents are various benzophenone derivatives having the general formula: wherein R through R₉ are each independently H, OH, NaO₃S, SO₃H, SO₃Na, Cl, R", OR" where R" is C₁₋₂₀ straight or branched chain alkyl Examples of such compounds include Benzophenone 1, 2, 3, 4, 5, 6, 7, 8, 9, 10, 11, and 12. Particularly preferred is where the benzophenone derivative is Benzophenone 3 (also referred to as Oxybenzone), Benzophenone 4 (also referred to as Sulisobenzone), Benzophenone 5 (Sulisobenzone Sodium), and the like. Most preferred is Benzophenone 3.

Also suitable are certain menthyl salicylate derivatives having the general formula: 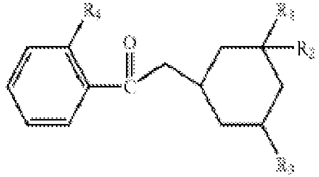 wherein R₁, R₂, R₃, and R₄ are each independently H, OH, NH₂, or C₁₋₂₀ straight or branched chain alkyl. Particularly preferred is where R₁, R₂, and R₃ are methyl and R₄ is hydroxyl or NH₂, the compound having the name homomenthyl salicylate (also known as Homosalate) or menthyl anthranilate. Homosalate is available commercially from Merck under the tradename Eusolex HMS and menthyl anthranilate is commercially available from Haarmann & Reimer under the tradename Heliopan. If present, the Homosalate should be found at no more than about 15% by weight of the total composition.

Various amino benzoic acid derivatives are suitable UVB absorbers including those having the general formula: 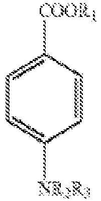 wherein R₁, R₂, and R₃ are each independently H, C₁₋₂₀ straight or branched chain alkyl which may be substituted with one or more hydroxy groups. Particularly preferred is wherein R₁ is H or C₁₋₈ straight or branched alkyl, and R₂ and R₃ are H, or C₁₋₈ straight or branched chain alkyl. Particularly preferred are PABA, ethyl hexyl dimethyl PABA (Padimate O), ethyldihydroxypropyl PABA, and the like. If present Padimate O should be found at no more than about 8% by weight of the total composition.

Salicylate derivatives are also acceptable UVB absorbers. Such compounds have the general formula: wherein R is a straight or branched chain alkyl, including derivatives of the above compound formed from mono-, di-, or triethanolamines. Particular preferred are octyl salicylate, TEA-salicylate, DEA-salicylate, and mixtures thereof.
Generally, the amount of the UVB chemical sunscreen present may range from about 0.001-45%, preferably 0.005-40%, more preferably about 0.01-35% by weight of the total composition.

If desired, the compositions of the invention may be formulated to have a certain SPF (sun protective factor) values ranging from about 1-50, preferably about 2-45, most preferably about 5-30. Calculation of SPF values is well known in the art. Preferably, the claimed compositions have SPF values greater than 4.

### E. Particulate Materials

The compositions of Embodiments I-V may contain particulate materials in the form of pigments, inert particulates, or mixtures thereof. If present, suggested ranges are from about 0.1-75%, preferably about 0.5-70%, more preferably about 0.1-65% by weight of the total composition. In the case where the composition may comprise mixtures of pigments and powders, suitable ranges include about 0.01-75% pigment and 0.1-75% powder, such weights by weight of the total composition.

### 1. Powders

The particulate matter may be colored or non-colored (for example white) non-pigmentatious powders. Suitable non-pigmentatious powders include bismuth oxychloride, titanated mica, fumed silica, spherical silica, polymethylmethacrylate, micronized teflon, boron nitride, acrylate copolymers, aluminum silicate, aluminum starch octenylsuccinate, bentonite, calcium silicate, cellulose, chalk, corn starch, diatomaceous earth, fuller's earth, glyceryl starch, hectorite, hydrated silica, kaolin, magnesium aluminum silicate, magnesium trisilicate, maltodextrin, montmorillonite, microcrystalline cellulose, rice starch, silica, talc, mica, titanium dioxide, zinc laurate, zinc myristate, zinc rosinate, alumina, attapulgite, calcium carbonate, calcium silicate, dextran, kaolin, nylon, silica silylate, silk powder, sericite, soy flour, tin oxide, titanium hydroxide, trimagnesium phosphate, walnut shell powder, or mixtures thereof. The above mentioned powders may be surface treated with lecithin, amino acids, mineral oil, silicone, or various other agents either alone or in combination, which coat the powder surface and render the particles more lipophilic in nature.

### 2. Pigments

The particulate materials may comprise various organic and/or inorganic pigments. The organic pigments are generally various aromatic types including azo, indigoid, triphenylmethane, anthroquinone, and xanthine dyes which are designated as D&C and FD&C blues, browns, greens, oranges, reds, yellows, etc. Organic pigments generally consist of insoluble metallic salts of certified color additives, referred to as the Lakes. Inorganic pigments include iron oxides, ultramarines, chromium, chromium hydroxide colors, and mixtures thereof. Iron oxides of red, blue, yellow, brown, black, and mixtures thereof are suitable.

### F. Preservatives

The compositions of Embodiments I-V may contain 0.001-8%, preferably 0.01-6%, more preferably 0.05-5% by weight of the total composition of preservatives. A variety of preservatives are suitable, including such as benzoic acid, benzyl alcohol, benzylhemiformal, benzylparaben, 5-bromo-5-nitro-1,3-dioxane, 2-bromo-2-nitropropane-1,3-diol, butyl paraben, phenoxyethanol, methyl paraben, propyl paraben, diazolidinyl urea, calcium benzoate, calcium propionate, caprylyl glycol, biguanide derivatives, phenoxyethanol, captan, chlorhexidine diacetate, chlorhexidine digluconate, chlorhexidine dihydrochloride, chloroacetamide, chlorobutanol, p-chloro-m-cresol, chlorophene, chlorothymol, chloroxylenol, m-cresol, o-cresol, DEDM Hydantoin, DEDM Hydantoin dilaurate, dehydroacetic acid, diazolidinyl urea, dibromopropamidine diisethionate, DMDM Hydantoin, and the like. In one preferred embodiment the composition is free of parabens.

### G. Vitamins and Antioxidants

The compositions of Embodiments I-V may contain vitamins and/or coenzymes, as well as antioxidants. If so, 0.001-10%, preferably 0.01-8%, more preferably 0.05-5% by weight of the total composition are suggested. Suitable vitamins include ascorbic acid and derivatives thereof, the B vitamins such as thiamine, riboflavin, pyridoxin, and so on, as well as coenzymes such as thiamine pyrophoshate, flavin adenin dinucleotide, folic acid, pyridoxal phosphate, tetrahydrofolic acid, and so on. Also Vitamin A and derivatives thereof are suitable. Examples are Vitamin A palmitate, acetate, or other esters thereof, as well as Vitamin A in the form of beta carotene. Also suitable is Vitamin E and derivatives thereof such as Vitamin E acetate, nicotinate, or other esters thereof. In addition, Vitamins D and K are suitable.

Suitable antioxidants are ingredients which assist in preventing or retarding spoilage. Examples of antioxidants suitable for use in the compositions of the invention are potassium sulfite, sodium bisulfite, sodium erythrobate, sodium metabisulfite, sodium sulfite, propyl gallate, cysteine hydrochloride, butylated hydroxytoluene, butylated hydroxyanisole, and so on.

### H. Film Formers

It may be desirable to include one or more film forming ingredients in the cosmetic compositions of the invention. Suitable film formers are ingredients that contribute to formation of a film on the keratinous surface. In some cases the film formers may provide films that provide long wearing or transfer resistant properties such that the cosmetic applied to the keratinous surface will remain for periods of time ranging from 3 to 16 hours. If present, such film formers may range from about 0.01 to 50%, preferably from about 0.1 to 40%, more preferably from about 0.5 to 35% by weight of the total composition. The film formers are most often found in the polymeric form and may be natural or synthetic polymers. If synthetic, silicone polymers, organic polymers or copolymers of silicones and organic groups may be acceptable.

### 1. Silicone Resins

One particularly suitable type of silicone film former is a silicone resin. Silicone resins are generally highly crosslinked structures comprising combinations of M, D, T, and Q units. The term "M" means a monofunctional siloxy unit having the general formula:

[Si-(CH₃)₃-O]_{0.5}

In cases where the M unit is other than methyl (such as ethyl, propyl, ethoxy, etc.) the M unit may have a prime after it, e.g. M'.

The term "D" means a difunctional siloxy unit having the general formula:

[Si-(CH₃)₂-O]_{1.0}

The difunctional unit may be substituted with alkyl groups other than methyl, such as ethyl, propyl, alkylene glycol, and the like, in which case the D unit may be referred to as D', with the prime indicating a substitution.

The term "T" means a trifunctional siloxy unit having the general formula:

[Si-(CH₃)-O]_{1.5}

The trifunctional unit may be substituted with substituents other than methyl, in which case it may be referred to as T'.

The term "Q" refers to a quadrifunctional siloxy unit having the general formula:

[Si-O-]_{2.0}

The silicone resins that may be used as film formers in the compositions of the invention preferably comprise highly crosslinked combinations of M, T, and Q units. Examples of such resins include trimethylsiloxysilicate which can be purchased from Dow Corning Corporation as 749 Fluid, or from GE Silicones under the SR-1000 tradename. Also suitable is a silicone resin that contains a large percentage of T groups, such as MK resin sold by Wacker-Chemie, having the CTFA name polymethylsilsesquioxane.

### 2. Copolymers of Silicone and Organic Monomers

Also suitable for use as the film formers are copolymers of silicone and organic monomers such as acrylates, methacrylates, and the like. Examples of such suitable film forming polymers include those commonly referred to as silicone acrylate or vinyl silicone copolymers, such as those sold by 3M under the brand name "Silicone Plus" polymers such as SA-70, having the CTFA name Polysilicone-7 and is a copolymer of isobutylmethacrylate and n-butyl endblocked polydimethylsiloxane propyl methacrylate; or VS-70 having the CTFA name Polysilicone-6, which is a copolymer of dimethylsiloxane and methyl-3 mercaptopropyl siloxane reacted with isobutyl methacrylate; or VS-80, having the CTFA name Polysilicone-8, which has the general structure: 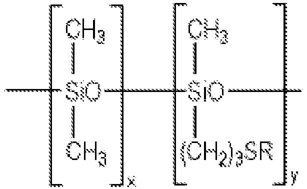 Where R represents the acrylates copolymer radical.

### 3. Organic Polymers

Also suitable as film formers include various types of organic polymers such as polymers formed from acrylic acid, methacrylic acid, or their simple C₁₋₁₀ carboxylic acid esters, such as methyl methacrylate, methyl acrylate, and the like.

### 4. Natural Polymers

Also suitable are various types of natural polymers such as shellac, natural resins, chitin, and the like.

### VII. The Cosmetic Compositions

The cosmetic compositions of Embodiment I may be found in a variety of forms, such as skin creams or lotions, or color cosmetic compositions such as foundation makeup, mascara, lip color, blush, eyeshadow, and the like. The resveratrol derivative may be found in the water phase or the oil phase of the emulsion depending on the type of derivative. For example, certain hydrophilic derivatives such as resveratrol triphosphate, resveratrol trisulfonate, and the like are water soluble and will generally be found in the aqueous phase of the emulsion or aqueous composition. Certain other derivatives are lipophilic in nature and will more likely be found in the oil phase of the emulsion.

Typical skin creams or lotions comprise from about 5-98% water, 1-85% oil, and from about 0.1 to 20% of one or more surfactants. Preferably the surfactants are nonionic and may be in the form of silicones or organic nonionic surfactants.

Typical aqueous based color cosmetic compositions such as foundations, blush, eyeshadow and the like will preferably contain from about 5-98% water, 1-85% oil, and from about 0.1 to 20% of one or more surfactants in addition to from about 0.1 to 65% of particulates that are pigments or a combination of pigments and powders.

Typical mascara compositions generally contain from about 5-98% water, 1-85% oil, and from about 0.1 to 20% surfactant in addition to natural or synthetic polymers that are film forming, such as aqueous dispersions of acrylic copolymers, aqueous dispersions of polyurethane, or silicone resins.

Typical color cosmetic compositions such as foundations, blush, eyeshadow, lipstick and the like will preferably contain from about 1-85% oil, from about 1 to 90% structuring agent, and from 0.1 to 65% of particulates that are pigments or a combination of pigments and powders. Optionally, the compositions may comprise from about 0.1 to 25% surfactant.

The compositions hereafter described illustrates some emulsion cosmetic compositions but are not part of the invention.

### EXAMPLE 1

Skin treatment oil-in- water (1), and oil-in- water-in-silicone oil (2), creams were prepared as follows:

| | w/w% | |
|---|---|---|
| Ingredient | 1 | 2 |
| Water | QS | QS |
| Hydroxyethyl urea | 0.50 | |
| Hyaluronic acid | 9.00 | 9.00 |
| Disodium EDTA | 0.12 | |
| Creatine | 0.05 | |
| Sucrose | 0.50 | |
| Caffeine | 0.20 | |
| Caprylyl glycol | 0.40 | 0.28 |
| Caprylic/capric triglyceride/cetyl alcohol/C12-20 acid PEG-8 ester | 4.00 | |
| PEG-100 stearate | 1.20 | |
| C12-20 acid PEG-8 ester | 4.96 | |
| Caprylic/capric triglyceride | 0.55 | |
| Behenyl alcohol | 0.50 | |
| Coco caprylate caprate | 5.10 | |
| Sweet almond oil | 0.10 | |
| Dimethicone, 100 cst. | 2.50 | |
| Dimethicone, 6 cst | | 5.00 |
| Dimethicone (silicone gum/20 cst dimethicone blend) | | 8.00 |
| Dimethicone/polysilicone 11 | | 6.00 |
| Dimethicone/dimethicone PEG-10/15 crosspolymer | | 1.00 |
| Lauryl PEG-9 polydimethylsiloxyethyl dimethicone | | 1.00 |
| Sesame oil | 0.10 | |
| Potassium cetyl phosphate | 0.50 | |
| Apricot kernel oil | 0.10 | |
| Wheat bran extract/olive extract | 0.20 | 0.20 |
| Cholesterol | 0.20 | |
| Linoleic acid | 0.20 | |
| Cholesterol/potassium sulfate | 0.20 | |
| Theobroma grandiflorum seed butter | 1.40 | |
| Lauryl PCA | 0.01 | 1.00 |
| Dimethicone | 1.50 | |
| Phenoxyethanol | 0.70 | 0.60 |
| Water/polyaminopropyl biguanide | 0.40 | |
| Glycerin | 2.00 | |
| Butylene glycol | 1.00 | |
| Hexylene glycol | | 0.05 |
| Mica/titanium dioxide | 1.00 | 0.75 |
| Mica/titanium dioxide/triethoxycaprylyl silane | | 0.50 |
| Pearl powder | 0.001 | |
| Silica | 0.50 | |
| 30% aqueous sodium hydroxide | 0.35 | |
| Trehalose | 0.50 | |
| N-acetyl glucosamine | 1.00 | 1.00 |
| Water/purified aribodopsis thaliana extract/lecithin | 0.50 | 1.00 |
| Aqueous solution acetyl hexapeptide-8 | 1.00 | 1.00 |
| Yeast ferment extract | 1.00 | 1.00 |
| Water/lecithin/micrococcus lysate | 0.50 | 0.50 |
| Milk protein/lactose/glucose/fructose | 0.50 | 0.50 |
| Saccharide isomerate | 0.50 | |
| Whey protein | 0.50 | 0.560 |
| Water/butylene glycol/lecithin/lauryldimonium hydroxypropyl hydrolyzed soy protein/lecithin/xanthan gum/ascorbyl tocopheryl maleate | 1.00 | 1.00 |
| Glycerin/padina povonica extract | 0.10 | 0.10 |
| Thermus thermophillus ferment/glycerin | 0.05 | |
| Camelina sativa seed oil | 0.05 | |
| Water/gold/hydrolyzed wheat protein | 0.001 | |
| Sorbitol/water/ascophyllum nodosum extract/asparagopsis armata extract | 0.25 | |
| Butylene glycol | 0.50 | |
| Boswellia serrata extract | 0.05 | |
| Calophyllum inophyllum (tamanu) seed oil | 0.05 | |
| Fragrance | 0.20 | |
| FD&C yellow No. 5 (1% aqueous solution) | 0.05 | |
| Aminomethyl propanol | | 0.03 |
| Sodim phosphate dibasic (10% aqueous solution) | | 0.75 |
| Citric acid (10% aqueous solution) | | 0.008 |
| Sodium acrylate/sodium acryloyldimethyl taurate copolymer/hydrogenated polydecene/laureth-8 | 1.00 | 1.00 |
| Ammonium acrylodimethyltaurate/VP copolymer | | 0.70 |
| Water/butylene glycol/decarboxy carnosine HCl | | 0.50 |
| Trisodium resveratrol triphosphate | 0.50 | 0.50 |

The composition was prepared by combining the water phase and oil phase ingredients separately, then emulsifying to form an emulsion.

### EXAMPLE 2

A water in silicone oil emulsion skin serum was prepared as follows:

| Ingredient | w/w% |
|---|---|
| Dimethicone/dimethicone PEG-10/15 crosspolymer | 4.00 |
| Dimethicone/dimethiconol | 1.00 |
| Dimethicone, 6 cst. | 6.00 |
| Trisiloxane (1.0 cst) | 16.00 |
| Water | QS |
| Phenoxyethanol | 0.50 |
| Caprylyl glycol/phenoxyethanol/hexylene glycol/iodopropynyl butylcarbamate | 0.50 |
| Water/polyaminobiguanide | 0.20 |
| Trisodium resveratrol triphosphate | 0.50 |
| Butylene glycol | 2.00 |
| Glycerin | 10.00 |
| Sodium citrate | 0.50 |

The composition was prepared by combining the oil phase ingredients and water phase ingredients separately, then mixing well to emulsify.

### EXAMPLE 3

Oil-in-water (O/W) and water-in-oil (W/O) emulsion mascaras were prepared as follows:

| Ingredient | w/w% | |
|---|---|---|
| | O/W | W/O |
| Ethylenediamine/Stearyl Dimer Tallate Copolymer - Uniclear 100VG, Arizona Chemical | 10. | 12.00 |
| PEG-30 Dipolyhydroxystearate | | 3.00 |
| Sorbitan tristearate | 1.00 | -- |
| Glyceryl stearate/PEG-100 stearate | 1.00 | -- |
| Stearic acid | 4.00 | 3.00 |
| Cetyl acetate/Acetylated lanolin alcohol | | 1.00 |
| Dioctyl adipate/octyl stearate/octyl palmitate | 1.00 | -- |
| Stearamide MEA stearate | 3.00 | -- |
| Glyceryl olivate | -- | 0.50 |
| Dioctyl malate | -- | 1.00 |
| Dimethicone | 2.50 | -- |
| Cyclomethicone | 5.00 | -- |
| Isododecane | 11.00 | 38.00 |
| Ethanol | 0.50 | -- |
| Water | QS | QS |
| Silica | 1.00 | -- |
| Polysorbate 20 | 2.00 | -- |
| Acacia gum | 0.25 | -- |
| Black iron oxide | 8.00 | 10.00 |
| Polyvinylpyrrolidone | 1.00 | -- |
| 4'-5' dihydroxystilbene-3-O-beta-mono-D-glucoside | 0.50 | -- |
| Shellac | 2.00 | -- |
| Acrylic copolymer solids dispersed in aqueous solution | 5.00 | 7.00 |
| Preservatives | 0.80 | -- |

The mascaras were made by combining the oily phase ingredients except for the cyclomethicone and dimethicone and heating to about 90° C. until solids melted. The cyclomethicone and dimethicone were added to the mixture and the heat maintained at about 60° C. The water phase ingredients were combined and heated to about 60° C. and combined with the mixture. The phases were emulsified to form the final mixture.

### EXAMPLE 4

Emulsion foundation makeup compositions were prepared as follows:

| Ingredient | w/w% |
|---|---|
| Cyclomethicone | 16.90 |
| Polysilicone-11 | 5.00 |
| Cyclomethicone/dimethiconol | 1.00 |
| Dimethicone copolyol | 1.50 |
| Sorbitan sesquioleate | 1.50 |
| Phenyl trimethicone | 10.00 |
| Dimethicone | 10.00 |
| Resveratrol tripalmitate | 0.50 |
| Red Iron Oxide treated with methicone | 0.50 |
| Yellow iron oxide treated with methicone | 1.22 |
| Black iron oxide treated with methicone | 0.13 |
| Titanium dioxide coated with methicone | 8.06 |
| Water | QS |
| Butylene glycol | 5.00 |
| Xanthan gum | 0.10 |
| Magnesium sulfate | 1.00 |
| Laureth-7 | 0.25 |

The water, oil and pigment phases were separately prepared by low shear mixing. The phases were combined with high shear blending to form a foundation makeup composition.

### EXAMPLE 5

Anhydrous emulsion skin treatment serums and gels were prepared as follows:

| Ingredient | w/w% | | |
|---|---|---|---|
| | A | B | C |
| Pinus pinaster bark extract | 0.50 | | |
| Santalum album (sandalwood) extract/phellodendron amurense bark extract | | | 6.00 |
| Calcium carbonate/zea mays (corn) | 0.5 | | |
| starch/glycine soja (soybean) extract | | | |
| Lauroyl lysine | | | 8.00 |
| Butylene glycol | 7.00 | 7.00 | 2.00 |
| Ferulic acid | 0.10 | | |
| Glycerin | 10.00 | 10.00 | |
| PEG-8 | | | 1.00 |
| Polysorbate-80 | | | 0.50 |
| Dicarylyl carbonate | | | 22.00 |
| PEG-60 hydrogenated castor oil | | | 1.00 |
| Simmondsia chinensis (jojoba) seed oil | | | 21.50 |
| Isopropyl isostearate | | | 5.00 |
| Isononyl isononanoate/ethylhexyl isononanoate | | | 17.00 |
| Dextrin palmitate | | | 7.00 |
| Glyceryl behenate/eicosadioate | | | 0.50 |
| Hinokitiol | 0.10 | | |
| PEG-10 dimethicone | 2.00 | 2.00 | |
| Dimethicone/caprylyl methicone/phenyl methicone | | 65.00 | |
| Silica | | | 7.50 |
| Nordihydroguaiaretic acid | 0.50 | | |
| Camellia sinensis (green tea) extract | 2.00 | | |
| Phenoxyethanol | 0.20 | 0.20 | 0.50 |
| Trisodium resveratrol phosphate | 0.50 | 0.50 | 0.50 |
| Cyclomethicone/dimethicone/C3 0-45 olefin/phenyl methicone | 2.00 | | |
| Methyl trimethicone | 3.00 | 3.00 | |
| Citrus grandis (grapefruit) peel extract | 0.20 | | |
| Dimethicone/vinyl dimethicone cross polymer/methyl trimethicone | 71.40 | 75.30 | |

The compositions were prepared by combining the resveratrol triphosphate and glycerin. The remaining ingredients were combined and mixed well, followed by addition of the resveratrol triphosphate in glycerin.

### EXAMPLE 6

A foundation makeup was prepared as follows:

| Ingredient | w/w% |
|---|---|
| Cetyl PEG/PPG-10/1 dimethicone/polyglyceryl-4 isostearate/hexyl laurate | 1.00 |
| Red iron oxide/methicone | 0.60 |
| Yellow iron oxide | 1.47 |
| Dimethicone crosspolymer-3/isododecane | 22.00 |
| Polyethylene | 4.00 |
| Mica | 10.37 |
| Trifluoromethyl C1-4 alkyl dimethicone/cyclomehticone/propylene carbonate | 6.00 |
| Titanium dioxide/methicone | 6.80 |
| Silica | 0.001 |
| Resveratrol tripalmitate | 0.50 |
| Cyclopentasiloxane | QS |
| Phenyl trimethicone | 3.80 |
| Dimethicone gum | 0.20 |
| Cyclomethicone/dimethicone/phenyl methicone | 28.10 |
| Mica/methyl methacrylate crosspolymer | 1.00 |
| Iron oxides/methicone | 0.20 |
| Titanium dioxide/iron oxides | 0.01 |
| Acrylates copolymer/diphenyl carbomethoxy acetoxy naphthopyran | 0.02 |
| Titanium dioxide/trimyristin/hydrogenated lecithin | 3.50 |

The composition was prepared by grinding the pigments in a portion of the oil. The remaining ingredient were combined with heat and mixed well, incorporating the pigment grind into the composition. The composition was a semi-solid beige colored composition suitable for use as a foundation.

### EXAMPLE 7

A lipstick composition was prepared as follows:

| Ingredient | w/w% |
|---|---|
| Aloe barbadensis extract/mineral oil | 0.50 |
| Trimethylsiloxypheny dimethicone (PDM 1000) | 1.00 |
| Octyldodecyl stearoyl stearate | 3.05 |
| Ceresin wax | 6.50 |
| Petrolatum | 32.05 |
| Hydrogenated vegetable oils | 14.00 |
| Polybutene | 0.25 |
| Ozokerite | 16.25 |
| Ethylhexyl methoxycinnamate | 7.50 |
| Propyl paraben | 0.15 |
| Phenyl trimethicone | 1.00 |
| Bis-diglyceryl polyacyladipate | 2.50 |
| Resveratrol triisostearate | 1.00 |
| Cetyl esters | QS |
| Ethylhexyl salicylate | 3.50 |
| Tocopheryl acetate | 0.25 |

The composition was prepared by grinding the pigments in a portion of the cetyl esters. The waxes and oils were separately combined with heat and mixed well. The pigment grind was added to the mixture and stirred well. The mixture was poured into molds and allowed to cool to room temperature.

### EXAMPLE 8

Powder eyeshadow and blush compositions were prepared as follows

| | w/w% | |
|---|---|---|
| Ingredient | Shadow | Blush |
| Aluminum hydroxide | 0.003 | |
| Sorbitan sesquioleate | 0.001 | |
| Ascorbyl palmitate | 0.04 | |
| Barium sulfate | 0.0005 | |
| Soybean extract | 2.47 | |
| BHT | 0.70 | 0.05 |
| Lecithin | | 0.0004 |
| Candelilla wax | 5.85 | |
| Carnauba | 1.76 | |
| Castor seed oil | QS | |
| Polyglyceryl-3 beeswax | 3.23 | |
| Simethicone | | 0.05 |
| Dipentaerythrityl hexahydroxystearate | 2.50 | |
| Isodecyl neopentanoate | 0.05 | |
| Caprylic/capric triglycerides | 9.90 | |
| Mica | 4.75 | |
| Oleyl oleate | 6.70 | |
| Octyl palmitate | | 7.00 |
| Polybutene | | QS |
| Hydrogenated polyisobutene | | 30.13 |
| Dextrin palmitate | | 11.00 |
| Ozokerite | 2.35 | |
| Synthetic wax | 4.95 | |
| Diisostearyl malate | 8.70 | |
| Bis-diglyceryl polyacyladipate-2 | 1.47 | |
| Polydecene | 2.10 | 0.35 |
| Mica/titanium dioxide | | 0.80 |
| Propyl paraben | | 0.10 |
| Titanium dioxide | 3.10 | |
| Tocopheryl acetate | 0.04 | |
| Iron oxides | 5.11 | |
| FD&C blue no. 1 aluminum lake | 0.10 | 0.002 |
| D&C Red No. 6 | | 0.01 |
| D&C Red No. 7 Calcium Lake | 0.36 | 0.25 |
| Fragrance | | 0.50 |
| 3,5,4'-triferulate stilbene | 0.80 | |
| 3,5-dimethoxy-4'-hydroxystilbene (Pterostilbene) | | 0.50 |

The compositions were prepared by grinding the pigments in a portion of the oil. Separately, the oils and waxes were combined with heat and mixed well. The pigment grind was added. The compositions were pressed into pans.

While the invention has been described in connection with the preferred embodiment, it is not intended to limit the scope of the invention to the particular form set forth but, on the contrary, it is intended to cover such alternatives, modifications, and equivalents as may be included within the scope of the invention as defined by the appended claims.

## Claims

1. An emulsion cosmetic composition comprising at least one ester of a resveratrol derivative selected from the group consisting of 3-salicylate-5,4'-dihydroxystilbene; 5-salicylate-3,4'-dihydroxystilbene; 4'-salicylate-3,5-dihydroxystilbene; 3,5-disalicylate-4'-hydroxystilbene; 3,4'-disalicylate-5-hydroxystilbene; 4',5-disalicylate-3-hydroxystilbene; 3,5,4'-trisalicylate stilbene and an aqueous phase, and an oil phase having at least one oil phase structuring agent.

2. The composition of claim 1 further comprising an oil phase structuring agent which is a silicone gum, non-emulsifying silicone elastomer, or silicone wax.

3. The composition of claim 1 further comprising at least one non-volatile silicone.

4. The composition of claim 1 further comprising at least one aqueous phase structuring agent.

5. The composition of claim 4 wherein the aqueous phase structuring agent comprises a polysaccharide, acrylate polymer, high molecular weight PEG, polyglycerin, or mixtures thereof.

6. The composition of claim 1 further comprising at least one silicone surfactant.

7. The composition of claim 6 wherein the silicone surfactant comprises at least one of dimethicone copolyols, alkyl dimethicone copolyols, and emulsifying silicone elastomers.

8. The composition of claim-7 further comprising at least one organic nonionic surfactant.

9. The composition of claims 1 further comprising one or more humectants.

## Patentansprüche

1. Kosmetische Emulsionszusammensetzung, die mindestens einen Ester eines Resveratrol-Derivats, ausgewählt aus der Gruppe, bestehend aus 3-Salicylat-5,4'-dihydroxystilben; 5-Salicylat-3,4'-dihydroxystilben; 4'-Salicylat-3,5-dihydroxystilben; 3,5-Disalicylat-4'-hydroxystilben; 3,4'-Disalicylat-5-hydroxystilben; 4',5-Disalicylat-3-hydroxystilben; 3,5,4'-Trisalicylatstilben, sowie eine wässrige Phase und eine Ölphase umfasst, die mindestens ein Strukturierungsmittel für die Ölphase aufweist.

2. Zusammensetzung nach Anspruch 1, weiter umfassend ein Strukturierungsmittel für die Ölphase, das ein Silikonkautschuk, ein nicht emulgierendes Silikonelastomer oder ein Silikonwachs ist.

3. Zusammensetzung nach Anspruch 1, weiter umfassend mindestens ein nicht flüchtiges Silikon.

4. Zusammensetzung nach Anspruch 1, weiter umfassend mindestens ein Strukturierungsmittel für die wässrige Phase.

5. Zusammensetzung nach Anspruch 4, wobei das Strukturierungsmittel für die wässrige Phase ein Polysaccharid, ein Acrylatpolymer, ein PEG mit hohem Molekulargewicht, ein Polyglycerin oder Gemische davon umfasst.

6. Zusammensetzung nach Anspruch 1, weiter umfassend mindestens ein Silikontensid.

7. Zusammensetzung nach Anspruch 6, wobei das Silikontensid mindestens eines von Dimethicon-Copolyolen, Alkyldimethicon-Copolyolen und emulgierenden Silikonelastomeren umfasst.

8. Zusammensetzung nach Anspruch 7, weiter umfassend mindestens ein organisches nicht ionisches Tensid.

9. Zusammensetzung nach Anspruch 1, weiter umfassend ein oder mehrere Feuchthaltemittel.

## Revendications

1. Composition cosmétique en émulsion comprenant au moins un ester d'un dérivé de resvératrol sélectionné dans le groupe constitué du 3-salicylate-5,4'-dihydroxy-stilbène ; du 5-salicylate-3,4'-dihydroxystilbène ; du 4'-salicylate-3,5-dihydroxystilbène ; du 3,5-disalicylate-4'-hydroxystilbène ; du 3,4'-disalicylate-5-hydroxystilbène ; du 4',5-disalicylate-3-hydroxystilbène ; du 3,5,4'-trisalicylate stilbène et une phase aqueuse, et une phase huileuse ayant au moins un agent de structuration de phase huileuse.

2. Composition selon la revendication 1 comprenant en outre un agent de structuration de phase huileuse qui est une gomme de silicone, un élastomère de silicone non émulsionnant, ou une cire de silicone.

3. Composition selon la revendication 1 comprenant en outre au moins une silicone non volatile.

4. Composition selon la revendication 1 comprenant en outre au moins un agent de structuration de phase aqueuse.

5. Composition selon la revendication 4 dans laquelle l'agent de structuration de phase aqueuse comprend un polysaccharide, un polymère d'acrylate, un PEG de poids moléculaire élevé, une polyglycérine, ou leurs mélanges.

6. Composition selon la revendication 1 comprenant en outre au moins un tensioactif à base de silicone.

7. Composition selon la revendication 6 dans laquelle le tensioactif à base de silicone comprend au moins l'un parmi les diméthicone copolyols, les alkyl diméthicone copolyols, et les élastomères émulsionnants à base de silicone.

8. Composition selon la revendication 7 comprenant en outre au moins un tensioactif organique non ionique.

9. Composition selon la revendication 1 comprenant en outre un ou plusieurs humectants.
